# EUROPÄISCHE PATENTSCHRIFT

(11) **EP 0 906 899 B1**
(45) Veröffentlichungstag und Bekanntmachung des Hinweises auf die Patenterteilung: **29.08.2001**
(21) Anmeldenummer: 98118032.6
(22) Anmeldetag: 23.09.1998
(51) Int. Cl.: C07C 41/42, C07C 43/16

(54) **Verfahren zur Trennung des Divinylether von Diethylenglycol oder Triethylenglycol vom Monovinylether des entsprechenden Oligoethylenglycol**
Process for the separation of the divinylether of diethylenglycol or triethylenglycol from the monovinylether of the corresponding oligoethylenglycol
Procédé de séparation du divinylether du diethyleneglycol du monovinylether de l'oligoethyleneglycol correspondant

(30) Priorität: 30.09.1997 DE 19743144
(43) Veröffentlichungstag der Anmeldung: 07.04.1999
(73) Patentinhaber: BASF AKTIENGESELLSCHAFT, 67056 Ludwigshafen (DE)
(72) Erfinder: Lorenz, Rudolf Erich, 67069 Ludwigshafen (DE)

(56) Entgegenhaltungen:
- US-A- 1 959 927
- PATENT ABSTRACTS OF JAPAN vol. 12, no. 270 (C-515), 27. Juli 1988 & JP 63 051344 A (DAICEL CHEM IND LTD), 4. März 1988

## Beschreibung

Vorliegende Erfindung betrifft ein Verfahren der destillativen Trennung des Divinylether von Diethylenglycol oder Triethylenglycol vom Monovinylether des entsprechenden Oligoethylenglycol.

Divinylether von Diolen sind gebräuchliche Vernetzer, die zur Erhöhung der inneren Festigkeit von durch radikalisch initiierte Polymerisation von wenigstens eine ethylenisch ungesättigte Gruppe aufweisenden Verbindungen (Monomere) hergestellten Polymeren Verwendung finden.

Die wichtigste großtechnische Methode zur Herstellung von Vinylethern ist die Addition von Alkohol an Acetylen im Beisein von katalytisch wirkendem Alkalialkoholat in homogener flüssiger Phase. Die vinylierungstemperaturen liegen in der Regel bei 150 bis 180°C. Ferner erfolgt die Reaktion in der Regel bei überatmosphärischem Druck.

Patent Abstr. of Japan vol. 12, no. 270, 1988 and JP-A-63 051344 beschreiben eine Methode zur Gewinnung von Dialkylenglykolmonoethern durch Zusatz von Natriumperiodat und Rektifikation als Trennmethode.

Im Fall der Herstellung von Divinylethern wird als Alkohol ein Diol eingesetzt. Die Vinylierung erfolgt dann in zwei aufeinanderfolgenden Schritten. Zunächst wird im wesentlichen nur der Monovinylether des Diols gebildet. Selbiger reagiert dann zum Divinylether weiter.

Wird als Diol Diethylenglycol oder Triethylenglycol verwendet, wird die Umsetzung zum entsprechenden Divinylether in der Regel abgebrochen, bevor eine quantitative Divinyletherbildung erfolgt ist. Ursächlich dafür ist eine mit der Umsetzung einhergehende Viskositätszunahme, die ein weitergehendes Einpressen von Acetylen zunehmend erschwert.

Im Normalfall fällt daher bei der Herstellung des Divinylether von Diethylenglycol oder Triethylenglycol ein Reaktionsgemisch an, das neben einer überwiegenden Menge an Divinylether noch bis zu 5 Gew.-%, bezogen auf die Gemischmenge, des entsprechenden Monovinylethers enthalten kann. Bei einer beabsichtigten Verwendung der Divinylether als Vernetzer wirkt sich ein Monovinylethergehalt jedoch störend aus, weshalb eine Trennung des Divinylether vom Monovinylether angezeigt ist. Zur Lösung dieser Trennaufgabe bietet sich in einfacher Weise eine rektifikative Aufarbeitung des Reaktionsgemisches bei reduziertem Druck an.

Letzteres ist aufgrund der hohen Siedepunkte des Divinylether von Diethylenglycol bzw. Triethylenglycol erforderlich. Bemerkenswerterweise liegt die Siedetemperatur des Divinylether von Diethylenglycol bzw. Triethylenglycol unterhalb der Siedetemperatur des jeweiligen entsprechenden Monoether, was vermutlich darauf zurückzuführen ist, daß der Monovinylether noch zur Ausbildung von Wasserstoffbrücken befähigt ist.

Da die Siedepunktsdifferenz zwischen Monovinylether und Divinylether in den beiden vorgenannten Fällen nicht sehr ausgeprägt ist (Sdp. von Diethylenglycolmonovinylether = 90°C bei 6 mbar; Sdp. von Diethylenglycoldivinylether = 89°C bei 1300 Pa (13 mbar)) war davon auszugehen, daß es zur Bewältigung der rektifikativen Trennaufgabe einer leistungsstarken Kolonne bedarf. Überraschenderweise wurde jedoch gefunden, daß der Gehalt an Monovinylether von Diethylenglycol oder Triethylenglycol im am Kolonnenkopf als Destillat anfallenden Divinylether des entsprechenden Diols, unabhängig vom Rücklaufverhältnis und der Anzahl der theoretischen Trennstufen, im wesentlichen nicht unter 1 Gew.-%, bezogen auf das Destillat, gesenkt werden konnte.

Dies wird darauf zurückgeführt, daß der Divinylether und der Monovinylether offensichtlich ein Azeotrop bilden.

Die Aufgabe der vorliegenden Erfindung, ein Verfahren der destillativen Trennung des Divinylether von Diethylenglycol oder Triethylenglycol von geringen Mengen Monovinylether des entsprechenden Oligoethylenglycol zur Verfügung zu stellen wurde vor dem Hintergrund des oben genannten Befundes dadurch gelöst, daß man dem destillativ aufzutrennenden Gemisch vor der destillativen Trennung ein Metallhydroxid zusetzt. Vorzugsweise wird ein Alkalimetallhydroxid und/oder ein Erdalkalimetallhydroxid zugesetzt. Besonders bevorzugt ist ein Zusatz von Kaliumhydroxid und/oder Natriumhydroxid. Selbstverständlich kann aber auch Calciumhydroxid verwendet werden.

Die Herstellung von Triethylenglycoldivinylether oder von Diethylenglycoldivinylether kann demnach so erfolgen, daß man das entsprechende Oligoethylenglycol im Beisein von Alkalialkoholat des entsprechenden Diols (bezogen auf das Oligoethylenglycol in der Regel bis zu 3 Gew.-%) mittels Acetylen vinyliert (vorzugsweise bei 150 bis 180°C und bei überatmosphärischem Druck von bis zu 2000 Pa (20 bar)), bis der verbleibende Anteil an Monovinylether, bezogen auf das Reaktionsgemisch, ≥ 0,5 Gew.-% und ≤ 5 Gew.-% beträgt. Danach kann man dem Reaktionsgemisch entweder unmittelbar Metallhydroxid zusetzen und daraufhin den Divinylether aus dem Reaktionsgemisch heraus von noch enthaltenem Monovinylether destillativ und/oder rektifikativ abtrennen, oder man trennt vom Reaktionsgemisch zunächst die flüchtigen Bestandteile, die im wesentlichen aus Divinylether und aus Monovinylether bestehen, destillativ ab, setzt dem erhaltenen Destillat Metallhydroxid zu, um anschließend den Divinylether vom Monovinylether destillativ und/oder rektifikativ abzutrennen.

Die zuzusetzende Menge an Metallhydroxid wird in der Regel erfindungsgemäß so bemessen, daß sie in der Lage wäre, die etwa 1,2-fache molare Menge des enthaltenen Monovinylether zu neutralisieren. Mit Vorteil wird das Metallhydroxid erfindungsgemäß als wäßrige Lösung zugesetzt.

Von Bedeutung ist, daß das erfindungsgemäße Verfahren nicht notwendigerweise als Rektifikation auszuführen ist, sondern auch als einfache Destillation (nur eine theoretische Trennstufe), z.B. in einem Sambay-Dünnschichtverdampfer, durchgeführt werden kann.

Erfindungsgemäß sind so in einfacher Weise Divinylether von Triethylenglycol oder Diethylenglycol erhältlich, deren Gehalt an Monovinylether des entsprechenden Oligoethylenglycol ≤ 0,1 Gew.-% beträgt.

### Beispiele

A) 200 g eines Gemisches bestehend aus

| | |
|---|---|
| 197 g | Triethylenglycoldivinylether |
| 2,1 g | Triethylenglycolmonovinylether und |
| 1 g | herstellungsbedingter Nebenprodukte |

wurde mit 1,5 g einer 50 gew.-%igen wäßrigen Kaliumhydroxidlösung versetzt. Anschließend wurde das Gemisch bei einem Arbeitsdruck von 600 Pa (6 mbar) einer einfachen Destillation unterworfen, bis kein Destillat mehr überging (Siedetemperatur ≈ 105°C).
Das gewonnene Destillat wies folgende Zusammensetzung auf:

| | |
|---|---|
| 99,66 Gew.-% | Triethylenglycoldivinylether |
| 0,1 Gew.-% | Triethylenglycolmonovinylether |
| 0,24 Gew.-% | sonstiges. |

Der Destillationsrückstand betrug etwa 6 g. Er bestand aus einer hochviskosen Lösung, die zu 24 % ihres Gewichtes Triethylenglycolmonovinylether enthielt.

B) Ein Gemisch, dessen Zusammensetzung derjenigen des Gemisches in A) entsprach, wurde in einer Füllkörperkolonne der nachfolgenden Art (21 theoretische Stufen) rektifiziert. Der Kolonnendurchmesser betrug 1,38 m. Die Füllkörper bestanden aus Pallringen 3S (Material: Edelstahl) der Fa. Raschig. Die Füllkörpergesamtschüttung wies eine Hohe von 14,5 m (verteilt auf 5 Schüttbetten) auf. Der Druck am Kolonnenkopf wurde auf 600 Pa (6 mbar) eingestellt. Die Sumpftemperatur betrug 140 bis 150°C. Das rektifikativ zu trennende Gemisch wurde der Kolonne auf der Höhe des .....ten theoretischen Bodens zugeführt.
Das am Kolonnenkopf gewonnene Kondensat wies nachfolgende Zusammensetzung auf:

| | |
|---|---|
| 99 Gew.-% | Triethylenglycoldivinylether und |
| 1 Gew.-% | Triethylenglycolmonovinylether. |

Eine sich anschließende Rektifikation des Kondensats in einer entsprechenden Kolonne und unter entsprechenden Bedingungen ergab keine weitere Auftrennung.

## Patentansprüche

1. Verfahren der destillativen und/oder rektifikativen Trennung des Divinylether von Diethylenglycol oder Triethylenglycol aus einem Gemisch, das zu wenigstens 95 Gew.-% aus einem der beiden vorgenannten Divinylether und aus bis zu 5 Gew.-% des Monovinylethers des entsprechenden Oligoethylenglycol besteht, **dadurch gekennzeichnet**, daß man dem Gemisch vor und/oder während der destillativen und/oder rektifikativen Trennung ein Metallhydroxid zusetzt.

2. Verfahren zur Herstellung des Divinylether von Diethylenglycol oder Triethylenglycol durch Vinylierung von Diethylenglycol oder Triethylenglycol mit Acetylen in flüssiger Phase und im Beisein von Alkalialkoholat des entsprechenden Oligoethylenglycol bis der verbleibende Anteil an Monovinylether, bezogen auf das Reaktionsgemisch, ≥ 0,5 Gew.-% und ≤ 5 Gew.-% beträgt und anschließende destillative und/oder rektifikative Abtrennung des Divinylether vom Reaktionsgemisch, **dadurch gekennzeichnet,** daß man dem ≥ 0,5 Gew.-% und 5 5 Gew.-% Monovinylether enthaltenden Reaktionsgemisch vor und/oder während der destillativen und/oder rektifikativen Abtrennung ein Metallhydroxid zusetzt.

## Claims

1. A process for separating by distillation and/or rectification the divinyl ether of diethylene glycol or triethylene glycol from a mixture which consists of at least 95% by weight of one of the two abovementioned divinyl ethers and of up to 5% by weight of the monovinyl ether of the corresponding oligoethylene glycol, which comprises adding a metal hydroxide to the mixture before and/or during the separation by distillation and/or rectification.

2. A process for preparing the divinyl ether of diethylene glycol or triethylene glycol by vinylation of diethylene glycol or triethylene glycol with acetylene in the liquid phase and in the presence of alkali metal alkoxide of the corresponding oligoethylene glycol until the remaining content of monovinyl ether, based on the reaction mixture, is ≥ 0.5% by weight and ≤ 5% by weight and subsequent separation by distillation and/or rectification of the divinyl ether from the reaction mixture, which comprises adding a metal hydroxide to the reaction mixture, which comprises ≥ 0.5% by weight and ≤ 5% by weight of monovinyl ether, before and/or during the separation by distillation and/or rectification.

## Revendications

1. Procédé de séparation par distillation et/ou rectification de l'éther divinylique de diéthylèneglycol ou de triéthylèneglycol à partir d'un mélange qui se compose d'au moins 95 % en poids d'un des deux éthers divinyliques susmentionnés et jusqu'à 5 % en poids de l'éther monovinylique de l'oligoéthylèneglycol correspondant, **caractérisé en ce que** l'on ajoute un hydroxyde métallique au mélange avant et/ou pendant la séparation par distillation et/ou rectification.

2. Procédé pour la préparation de l'éther divinylique de diéthylèneglycol ou de triéthylèneglycol par l'intermédiaire de la vinylation du diéthylèneglycol ou du triéthylèneglycol avec l'acétylène, en phase liquide et en présence d'un l'alcoolate alcalin de l'oligoéthylèneglycol correspondant, jusqu'à ce que la partie restante d'éther monovinylique, rapporté au mélange réactionnel, soit ≥ 0,5% en poids et ≤ 5% en poids, et la séparation suivante par distillation et/ou par rectification de l'éther divinylique du mélange réactionnel, **caractérisé en ce que** l'on ajoute, avant et/ou pendant la séparation par distillation et/ou rectification, un hydroxyde métallique au mélange réactionnel contenant ≥ 0,5 % en poids et ≤ 5% en poids d'éther monovinylique.
